# EUROPÄISCHE PATENTSCHRIFT

(11) **EP 4 208 125 B1**
(45) Veröffentlichungstag und Bekanntmachung des Hinweises auf die Patenterteilung: **09.10.2024**
(21) Anmeldenummer: 20775581.0
(22) Anmeldetag: 04.09.2020
(51) Int. Cl.: A61F 2/16

(54) **INJEKTOR FÜR INTRAOKULARLINSEN**
INJECTOR FOR INTRAOCULAR LENSES
INJECTEUR POUR LENTILLE INTRAOCULAIRE

(43) Veröffentlichungstag der Anmeldung: 12.07.2023
(73) Patentinhaber: OPHTHALMO Pro GmbH, 66386 Sankt Ingbert (DE)
(72) Erfinder: BAYER, Alexander, 40472 Düsseldorf (DE)
(74) Vertreter: Walther Bayer Faber Patentanwälte PartGmbB
(86) Internationale Anmeldenummer: PCT/EP2020/074761
(87) Internationale Veröffentlichungsnummer: WO 2022/048766

(56) Entgegenhaltungen:
- WO-A1-2019/195951
- US-A- 4 810 249
- US-A1- 2016 128 752
- US-B2- 8 105 332
- US-B2- 9 326 848

## Beschreibung

Die Erfindung betrifft einen Injektor zur Implantation einer Intraokularlinse in ein menschliches Auge, aufweisend einen Grundkörper, in den rückseitig ein länglich ausgebildetes Betätigungselement abschnittsweise hineinragt und das in einer Betätigungsachse beweglich geführt ist, und wobei eine Bewegung des Betätigungselementes in den Grundkörper wahlweise mittels einer Schubbetätigung entlang der Betätigungsachse oder mittels einer Schraubbetätigung um die Betätigungsachse herum erzeugbar ist, und wobei das Betätigungselement wenigstens auf einem Abschnitt ein Außengewinde aufweist, und wobei am Grundkörper ein um die Betätigungsachse drehbares Stellelement mit einem Durchgang angeordnet ist, durch den sich das Betätigungselement hindurch erstreckt.

### STAND DER TECHNIK

Injektoren zur Implantation von Intraokularlinsen in ein menschliches Auge sind hinreichend bekannt. Eine Intraokularlinse kann hierfür entweder über eine Linsenkartusche mit einer in dieser eingebrachten Intraokularlinse kurz vor dem Einsetzen in das Auge in den Injektor eingebracht werden, um nach anschließender Zugabe eines Viskoelastikums die Intraokularlinse aus einer vorderseitigen Austriebsdüse in die hintere Augenkammer des Auges auszutreiben. Die Austriebsdüse bildet das offene Ende eines Linsenführungsteils des Injektors, der vorderseitig am Grundkörper angeordnet ist.

Im Grundkörper ist ein Kolben entlang der Betätigungsachse beweglich geführt, und der Kolben, der mit dem Betätigungselement in der Betätigungsachse voreinander liegend in Verbindung steht, kann bei einem Vorschub des Betätigungselementes und damit übertragen auf den Kolben in Richtung zur Austriebsdüse mit der Intraokularlinse in Kontakt gelangen und diese durch die Austriebsdüse austreiben.

Um den Kolben innerhalb des Grundkörpers des Injektors und weiter voranschreitend in Richtung zur Austriebsdüse auch durch das Linsenführungsteil hindurch kontrolliert zu bewegen, ist das Betätigungselement vorgesehen, das so in den Grundkörper hineinragt, dass bei einem Vorschub des Betätigungselementes in der Betätigungsachse der Kolben erst die Intraokularlinse kontaktiert und im weiteren Verlauf gemeinsam mit der Intraokularlinse in Richtung zur Austriebsdüse bewegt werden kann, bis die Intraokularlinse in die hintere Augenkammer ausgetrieben ist. Injektoren der neueren Bauart werden als Einweginjektoren verwendet und nach dem einmaligen Gebrauch und dem Austrieb der voreingelegten Intraokularlinse entsorgt. Diese Bauart der Injektoren wird allgemein als Pre-Load System bezeichnet.

Das Einschieben des Betätigungselementes in eine hintere Öffnung im Grundkörper erfolgt dabei in der Regel manuell durch den Bediener, also dem Ophthalmologen. Dabei sind Injektoren bekannt, die in einem ersten Betriebsmodus eine reine Schubbewegung ermöglichen, die manuell in das Betätigungselement eingebracht wird, und in einem zweiten Betriebsmodus eine Drehbewegung ermöglichen, die in das gleiche Betätigungselement eingebracht wird, um damit das Betätigungselement entlang der Betätigungsachse zu bewegen. Insofern sind Injektoren neuerer Bauart in der Lage, sowohl eine Schubbetätigung als auch eine Schraubbetätigung zu erlauben, wofür am Injektor eine entsprechende Vorrichtung zur Umschaltung des Betriebsmodus vorgesehen wird.

Beispielsweise offenbart die EP 3 476 375 A1 einen Injektor zur Implantation einer Intraokularlinse, und das Betätigungselement kann entweder durch ein rückseitiges Schieben oder durch eine Schraubbewegung in den Grundkörper eingetrieben werden, wobei die Schraubbewegung manuell in ein Stellelement eingeleitet werden kann, das eine direkte Gewindeverbindung mit einem Außengewinde des Betätigungselementes aufweist. Wird das Betätigungselement hingegen manuell in den Grundkörper hineingedrückt, so dreht das Stellelement frei mit. Wird das Betätigungselement wahlweise durch rückseitiges Drücken auf das Betätigungselement oder durch ein Drehen des Stellelementes in den Grundkörper hineingetrieben, so kann dieses in Kontakt mit dem Kolben die Intraokularlinse vorderseitig aus der Austriebsdüse austreiben.

Die WO 2019/195951 A1 offenbart eine weitere Ausgestaltung eines Injektors zur Implantation einer Intraokularlinse in ein menschliches Auge, aufweisend einen Grundkörper, in den rückseitig ein länglich ausgebildetes Betätigungselement abschnittsweise hineinragt und in einer Betätigungsachse beweglich geführt ist, und wobei eine Bewegung des Betätigungselementes in den Grundkörper wahlweise mittels einer Schubbetätigung des Betätigungselementes entlang der Betätigungsachse oder mittels einer Schraubbetätigung des Betätigungselementes um die Betätigungsachse erzeugbar ist, wofür das Betätigungselement wenigstens auf einem Abschnitt ein Außengewinde aufweist. Um zwischen der Schubbetätigung und der Schraubbetätigung des Betätigungselementes umzuschalten, sind zwei sich gegenüberliegende ein- und ausklappbare Flügelgriffe vorgesehen, wobei durch die ausgeklappte Position der Betriebsmodus auf eine Schubbetätigung und in einer eingeklappten Position auf eine Schraubbetätigung umgeschaltet werden kann. Im Betriebsmodus der Schubbetätigung können dabei die ein- und ausklappbaren Flügelgriffe als Fingergriffe dienen, sodass ein Operateur den Injektor an den Flügelgriffen zwischen Zeigefinger und Mittelfinger greifen kann, um schließlich mit dem Daumen rückseitig das Betätigungselement in den Grundkörper hineinzuschieben. Sind die Flügelgriffe eingeklappt, kann das Betätigungselement in den Grundkörper eingeschraubt werden, indem der Grundkörper in einer ersten Hand des Operateur gehalten wird und das Betätigungselement mit der zweiten Hand des Operateurs eingeschraubt wird.

Ein weiterer Injektor ist aus der US 8,105,332 B2 bekannt, der ein am Grundkörper angeordnetes und um die Betätigungsachse drehbares Stellelement mit einem Durchgang aufweist, durch den sich ein Betätigungselement hindurch erstreckt. Dabei wirkt das Stellelement mit losen Kugeln zusammen, die mit einer Innenkontur des Stellelementes in Kontakt sind, wobei die Innenkontur einen veränderlichen Radius aufweist. Wird das Stellelement um die Betätigungsachse verdreht, so laufen die Kugeln gemäß einer Freigabeposition entweder außenseitig auf den Grundkörper auf oder diese werden gemäß einer Eingriffsposition radial nach innen in Öffnungen hinein gedrückt, die im Grundkörper eingebracht sind. In der Freigabeposition kann das Betätigungselement frei durch den Grundkörper hindurchlaufen und in der Eingriffsposition greifen die Kugeln in ein Außengewinde auf dem Betätigungselement ein, sodass dieses nun in den Grundköper eingeschraubt werden kann.

Aus der US 2016/0128752 A1 ist eine Misch- und Ausgabeeinrichtung für Knochenzement oder künstliche Knochenersatzmasse bekannt, die einen austauschbaren Druckkolben aufweist, der mit einem Außengewinde ausgeführt ist. Wird der Druckkolben in den Grundkörper eingedrückt, so wirkt dieser mit am Grundkörper angeordneten Rastklinken zusammen. So kann der Druckkolben in den beispielsweise mit Knochenzement oder mit künstlicher Knochenersatzmasse gefüllten Grundkörper eingedrückt werden, während dieser durch die Rastklinken an einem erneuten Herauslaufen gehindert ist. Die Rastklinken federn dabei beim Überlauf über das Gewinde radial auf, sodass der Druckkolben zwar in den Grundkörper eingedrückt aber nicht mehr herausgezogen werden kann. Um bei größeren notwendigen Kräften zum Eindrücken des Druckkolbens und zum Austreiben der Mischung beispielsweise aus Knochenzement oder aus Knochenersatzmasse den Druckkolben mit einer Schraubbewegung einzutreiben, kann eine Sperrhülse axial auf die Rastklinken aufgedrückt werden, sodass diese nicht mehr radial auffedern und insofern ein Innengewindegang bilden, in den der Druckkolben mittels des Außengewindes eingeschraubt werden kann.

Schließlich offenbart die US 4,810,249 A eine medizinische Spritze mit einem Kolben, der ein Außengewinde aufweist, und wahlweise kann der Kolben der Spritze mit einer an sich bekannten Druckbewegung in den Grundkörper der Spritze eingedrückt werden oder es wird eine am Grundkörper vorhandene Klinke aktiviert, die nach der Aktivierung einen Eingriff in das Außengewinde des Kolbens bildet, sodass der Kolben in den Grundkörper nicht mehr eingedrückt, sondern nur noch eingeschraubt werden kann.

Die Einstellung des Injektors entweder für die Schubbetätigung oder für die Schraubbetätigung erfolgt in der Regel durch eine assistierende Person, sodass anschließend der Ophthalmologe den Injektor mit dem voreingestellten Betriebsmodus anwendet, indem die Austriebsdüse am vorderen Linsenführungsteil in den Augapfel eingeführt wird, und indem anschließend die Betätigung des Betätigungselementes erfolgt, bis die Intraokularlinse in die hintere Augenkammer eingetrieben wurde.

### OFFENBARUNG DER ERFINDUNG

Aufgabe der Erfindung ist die Weiterbildung eines Injektors zur Implantation einer Intraokularlinse in ein menschliches Auge, wobei der Injektor eine möglichst einfache Umschaltbarkeit zwischen der Schubbetätigung und der Schraubbetätigung des Betätigungselementes zum Vortrieb der Intraokularlinse aufweisen soll. Insbesondere soll eine Umschaltung des Betriebsmodus zwischen der Schubbetätigung und der Schraubbetätigung so möglich sein, dass der Injektor im Übrigen auf gleiche Weise zu benutzen ist, wobei jedoch die Umschaltung zwischen der Schubbetätigung und der Schraubbetätigung unmittelbar vor der Benutzung des Injektors einstellbar sein sollte.

Diese Aufgabe wird ausgehend von einem Injektor gemäß dem Oberbegriff des Anspruchs 1 in Verbindung mit den kennzeichnenden Merkmalen gelöst. Vorteilhafte Weiterbildungen der Erfindung sind in den abhängigen Ansprüchen angegeben.

Die Erfindung schließt die technische Lehre ein, dass am Grundkörper wenigstens ein Federarm angeordnet ist, der an einer zum Außengewinde weisenden Innenfläche eine Gewindeeingriffsstruktur aufweist, , wobei der Durchgang des Stellelementes wenigstens eine Innenoberfläche aufweist, die in einer Umfangsrichtung wenigstens abschnittsweise einen sich ändernden Innenradius aufweist, und wobei eine Außenfläche des Federarms mit der Innenoberfläche des Stellelementes in Kontakt steht, sodass bei einer Verdrehung des Stellelementes um die Betätigungsachse aufgrund des sich ändernden Innenradius an der Umfangsposition des Federarmes eine elastische Verformung des Federarms zur Betätigungsachse hin und von der Betätigungsachse weg erzeugbar ist, wodurch die Gewindeeingriffsstruktur mit dem Außengewinde wahlweise in Eingriff oder außer Eingriff bringbar ist.

Kerngedanke der Erfindung ist ein Stellelement, das manuell zwischen zwei Stellpositionen verdreht werden kann, und in einer ersten Stellposition bewirkt das Stellelement ein Eingreifen der Gewindeeingriffsstruktur am Federarm in das Außengewinde des Betätigungselementes für die Schraubbetätigung, und in der zweiten Stellposition gibt das Stellelement den Federarm mit der Gewindeeingriffsstruktur aus dem Außengewinde des Betätigungselementes frei, sodass dieses außer Eingriff mit dem Außengewinde am Betätigungselement gelangt und das Betätigungselement schließlich für die Schubbetätigung frei verschiebbar ist.

Wird also das Stellelement in die zweite Stellposition verdreht, so kann der Federarm oder können mehrere Federarme, insbesondere zwei sich gegenüberliegende Federarme, von selbst elastisch nach außen federn, sodass die Gewindeeingriffsstruktur das Außengewinde des Betätigungselementes freigibt.

Durch die Innenoberfläche mit dem sich über dem Umfang ändernden Innenradius wird bei einer Verdrehung des Stellelementes in eine erste Stellposition der wenigstens eine Federarm radial nach innen gedrückt, sodass die Gewindeeingriffsstruktur mit dem Außengewinde in Eingriff gelangt. Hierfür steht die Außenfläche des Federarms, insbesondere rückseitig der innen liegenden Gewindeeingriffsstruktur, mit der Innenoberfläche des Stellelementes in Kontakt, und die Innenoberfläche zieht sich bei einem Verdrehen des Stellelementes gewissermaßen zu, sodass der oder die Federarme nach innen gedrückt werden und bei einem Verdrehen des Stellelementes in entgegengesetztem Drehsinn wieder nach außen zurück federn. Die Federarme sind dabei in der Umfangsposition fest am Grundkörper angeordnet, und drehen mit der Drehung des Stellelementes insofern nicht mit.

Das Stellelement ist damit insbesondere gewindelos ausgeführt. Auf diese Weise kann der Injektor mit einem einfachen Stellelement in Form eines Drehringes oder eines Drehkranzes zwischen dem Betriebsmodus der Schubbetätigung und der Schraubbetätigung umgeschaltet werden, ohne dass außenseitig am Injektor ein Element verschoben oder umgeklappt werden muss. Das Stellelement kann beschriften sein, beispielsweise mit "Push" und "Screw", und es können Pfeile auf dem Stellelement aufgebracht sein, sodass der Benutzer unschwer erkennt, in welcher Richtung das Stellelement um die Betätigungsachse in Anordnung am Grundkörper verdreht werden muss, um wahlweise die Schubbetätigung oder die Schraubbetätigung zu aktivieren.

Der Kontakt zwischen dem Federarm und dem Stellelement muss im Rahmen einer möglichen erfindungsgemäßen Ausgestaltung kein direkter Kontakt sein, und es kann beispielsweise auch vorgesehen sein, dass ein Exzenterelement, insbesondere mit einem exzentrischen Innenteil, zwischen dem Stellelement und dem Federarm angeordnet ist, um im Sinne der Erfindung die gleiche Wirkung zu erzielen, oder das Stellelement ist mehrteilig ausgebildet. Dabei kann auch ein Exzenterelement mit Bezug auf eine Rotierbarkeit um die Betätigungsachse einen sich ändernden Innenradius erzeugen, in dessen Kontakt der wenigstens eine Federarm steht, sodass der gleiche Effekt erzielt wird wie mit dem Stellelement, an dem selbst die Innenoberfläche mit dem sich in Umfangsrichtung ändernden Innenradius ausgebildet ist.

Mit besonderem Vorteil weist der Grundkörper einen Aufnahmeabschnitt mit einem Durchgang auf, durch den sich das Betätigungselement hindurch erstreckt und auf den das Stellelement verdrehbar aufgenommen ist. Der Aufnahmeabschnitt kann dabei einteilig mit dem Grundkörper ausgebildet sein oder der Aufnahmeabschnitt ist an den Grundkörper angebracht, beispielsweise kann dieser an diesem verklipst sein.

Auch ist es vorteilhaft, wenn der wenigstens eine Federarm endseitig am oder innerhalb des Aufnahmeabschnittes am Aufnahmeabschnitt selbst oder alternativ am Grundkörper angeordnet ist. Der Federarm kann beispielsweise mit einem Abschnitt durch den hohl ausgeführten Aufnahmeabschnitt hindurch verlaufen und rückseitig aus einem offenen Ende des Aufnahmeabschnittes herausragen. So kann auf einfache Weise ein Teil der Innenoberfläche des Stellelementes mit der Außenfläche des Federarms in Kontakt gebracht werden, während ein weiterer Teil der Innenoberfläche mit einem über dem Umfang gleich bleibenden Innenradius ausgeführt ist, mit dem das Stellelement auf dem Aufnahmeabschnitt verdrehbar aufgenommen ist.

Mit weiterem Vorteil ist das Stellelement auf dem Aufnahmeabschnitt aufgerastet und/oder in einer mit Bezug auf die Betätigungsachse festen Axialposition auf dem Aufnahmeabschnitt um eine eigene Rotationsachse, die mit der Betätigungsachse zusammenfällt, verdrehbar geführt. Das Stellelement kann im Herstellungsprozess auf den Aufnahmeabschnitt aufgerastet werden, sodass das Stellelement mit einer ringförmigen Ausgestaltung in einer festen Axialposition auf dem Aufnahmeabschnitt um die Betätigungsachse drehbeweglich geführt ist. Das Stellelement kann dabei so auf dem Aufnahmeabschnitt angeordnet sein, dass dieses nicht manuell vom Aufnahmeabschnitt wieder entnommen werden kann, indem auf dem Außenumfang des Aufnahmeabschnittes beispielsweise Rastmittel vorgesehen sind, über die das Stellelement nur in einer Aufrastrichtung bewegt werden kann und die das Stellelement in einer entgegengesetzten Entnahmerichtung zurückhalten.

Ferner können Rastpositionen für das Stellelement in Umfangsrichtung vorgesehen sein, die so ausgeführt sind, dass der Benutzer eine haptische Rückmeldung erfährt, beispielsweise indem vor der Rastposition ein Widerstand überwunden werden muss, und sodass das Stellelement in der Rastposition für den Schubbetrieb und in der Rastposition für den Schraubbetrieb jeweils selbsthaltend verrasten kann, etwa in seinen rotatorischen Endlagen.

Mit weiterem Vorteil weist der Grundkörper einen Flügelgriff auf, wobei sich der Aufnahmeabschnitt rückseitig des Flügelgriffs als Fortsatz an diesen anschließt. Benutzt der Operateur den Injektor entweder im Schubmodus oder im Schraubmodus, so stört das Stellelement bei der Handhabung des Injektors nicht, da sich dieses hinter dem Flügelgriff befindet und während das Betätigungselement entweder mittels einer Schubbewegung oder mittels einer Schraubbewegung in den Grundkörper rückseitig eingetrieben wird, kann der Benutzer das Stellelement nicht verstellen, insbesondere nicht versehentlich.

Schließlich ist es von Vorteil, dass im Außenumfang des Aufnahmeabschnittes wenigstens eine Nut eingebracht ist, und wobei innenseitig im Stellelement wenigstens ein Zapfen ausgebildet ist, der in der Nut geführt ist. Die Nut ist insbesondere wenigstens auf einem Abschnitt in Umfangsrichtung der Außenoberfläche des Aufnahmeabschnittes über einen Teilumfang ausgebildet, wobei auch zwei Nuten mit jeweils zugeordneten innenseitig im Stellelement ausgebildeten Zapfen vorgesehen sein können, sodass das Stellelement verkippungsfrei auf dem Betätigungselement aufgebracht ist. Insbesondere kann jeder Nut auch mehr als ein Zapfen zugeordnet sein. Auch ist es von Vorteil, dass das Stellelement einen Innenumfangsabschnitt mit einer gewissen axialen Breite aufweist, der auf der Außenoberfläche des Aufnahmeabschnittes geführt wird, sodass eine kippelfreie, spielminimale und haptisch hochwertige Verdrehbarkeit des Stellelementes erreicht wird.

Die Nut kann auf wenigstens einem Abschnitt in Umfangsrichtung der Außenoberfläche des Aufnahmeabschnittes verlaufen. Um das Stellelement auf den Aufnahmeabschnitt aufzuschieben, kann eine parallel zur Betätigungsachse verlaufende weitere Nut vorgesehen sein, die in die umlaufene Nut übergeht. Die Nut oder die Nuten sind für jeden Zapfen auf der Innenseite des Stellelementes vorgesehen, wodurch die Montage des Stellelementes vorteilhaft ermöglicht wird. Der Übergang der axial verlaufenden Nut in die in Umfangsrichtung verlaufende Nut kann eine Rastschwelle aufweisen, über die der Zapfen hinweggedrückt werden muss, und sodass das Stellelement schließlich verliersicher auf dem Aufnahmeabschnitt angebracht werden kann.

Der sich in Umfangsrichtung ändernde Innenradius auf der Innenoberfläche des Stellelementes kann insbesondere als ein Abschnitt 1-fach oder mehrfach ausgestaltet sein. Insbesondere können ein oder mehrere Umfangssegmente eingerichtet sein, sodass die Innenoberfläche mit dem sich in Umfangsrichtung ändernden Innenradius auf einem Umfangssegment des Stellmittels ausgebildet ist. Dabei können auch über dem Umfang insbesondere gleichverteilt mehrere Umfangssegmente eingerichtet sein, beispielsweise zwei sich gegenüberliegende Umfangssegmente mit jeweils sich ändernden Innenradien.

Der sich ändernde Innenradius der Innenoberfläche erzeugt einen abhängig von der Umfangsposition des Stellelementes kleiner oder größer werdenden Abstand der Innenoberfläche zur Betätigungsachse, und wird das Stellelement auf dem Aufnahmeabschnitt verdreht, so zieht sich die Innenoberfläche an einer festen Umfangsposition, an der ein Federarm angeordnet ist, in gewisser Weise zu und weitet sich in entgegengesetzter Drehrichtung wieder auf, sodass dadurch der oder die Federarme nach innen und selbst-elastisch wieder nach außen bewegbar sind, wodurch schließlich die Gewindeeingriffsstruktur mit dem Außengewinde auf dem Betätigungselement in Eingriff und außer Eingriff gebracht werden kann.

In axialer Richtung des Stellelementes, also in Richtung einer Rotationsachse oder Symmetrieachse, in der das Stellelement am Grundkörper verdreht werden kann, kann für eine diskrete Umfangsposition der Innenradius dabei selbstverständlich konstant bleiben. Somit ergibt sich insbesondere eine Linienberührung zwischen der Innenoberfläche und der Außenfläche des Federarms, wobei die Außenfläche des Federarms auch so gekrümmt sein kann, dass diese an die gekrümmte Innenoberfläche mit dem sich ändernden Innenradius angepasst ist, sodass sich schließlich auch eine Flächenberührung zwischen der Außenfläche des Federarms und der Innenoberfläche mit dem sich nach innen zuziehenden Innenradius ergeben kann.

Das Umfangssegment kann sich auf einem Umfang mit einem Umfangswinkel von 360° oder auf einem Umfangswinkel von 180° oder auf einem Umfangswinkel von 120° oder auf einem Umfangswinkel von 90° erstrecken. Somit kann beispielsweise nur ein Federarm in Kontakt mit der Innenoberfläche stehen, wenn sich der Umfangswinkel über dem Vollkreis erstreckt, bei einem Umfangswinkel von 180° des Umfangssegmentes sind zwei sich gegenüberliegende Innenoberflächen vorgesehen, denen insofern zwei Federarme zugeordnet sind, und wenn der Umfangswinkel des Umfangssegmentes 120° beträgt, sind drei Umfangssegmente vorgesehen, denen drei Federarme zugeordnet sind und bei einem Umfangswinkel von 90° eines jeden Umfangssegmentes können vier Umfangssegmente vorgesehen werden, denen vier Federarme zugeordnet sind, und so weiter.

Insbesondere und bevorzugt können zwei sich gegenüberliegende Umfangssegmente in der Innenoberfläche des Stellelementes ausgebildet sein, sodass sich auch zwei gegenüberliegende Federarme am Grundkörper und/oder am Aufnahmeelement befinden, die jeweils einem Umfangssegment zugeordnet sind und mit diesen zusammenwirken. Wird der Injektor zwischen der Schubbetätigung und der Schraubbetätigung verstellt, so kann das Stellelement auf knapp 180° verdreht werden, wobei die Breite des Federarms zu berücksichtigen ist, sodass sich keine vollen 180° als Verdrehwinkel ergeben. Der Verdrehwinkel zur Verstellung zwischen den Betriebsmodi des Injektors beträgt insofern regelmäßig weniger als die Umfangserstreckung der Umfangssegmente mit den zugeordneten Umfangswinkeln.

Weiterhin ist es von Vorteil, wenn das Außengewinde auf dem Betätigungselement eine Steigung von 3mm bis 20mm oder von 5mm bis 15mm oder von 9mm bis 11mm aufweist. Insbesondere ist es vorteilhaft, wenn das Außengewinde auf dem Betätigungselement eine Steigung von 10mm aufweist, sodass der Operateur bei der Schraubbetätigung eine angemessene Anzahl von Umdrehungen in das Betätigungselement einleiten muss, um die Intraokularlinse vorteilhaft austreiben zu können, insbesondere um nicht zu viele Umdrehungen und nicht zu wenige Umdrehungen ausführen zu müssen, da bei letzteren eine feine Dosierung der Austriebsbewegung der Linse aus der Austriebsdüse verloren gehen würde.

Der Injektor kann ein Aufnahmemittel für eine Linsenkartusche aufweisen, in der eine Intraokularlinse eingebracht ist und wobei die Linsenkartusche mit der Intraokularlinse in das Aufnahmemittel einsetzbar ist. Derartige Injektoren können unabhängig von der Spezifikation der Intraokularlinse angeboten werden, wobei jedoch zur Vorbereitung der Anwendung des Injektors die Linsenkartusche eingesetzt werden muss, die die Intraokularlinse mit der erforderlichen Spezifikation für den Patienten enthält. Anschließend muss ein Viskoelastikum eingespritzt werden, sodass die Intraokularlinse und auch das innere Lumen in der Linsenkartusche sowie der Innenraum des Linsenführungsteils mit Viskoelastikum benetzt und ausgefüllt sind. Schließlich kann der Austrieb der Linse folgen, wobei der Injektor mehrfach verwendbar oder vorzugsweise als EinmalVerwendung vorgesehen ist.

Weiterhin kann der Injektor als sogenanntes Pre-Load-System ausgeführt sein, wonach der Grundkörper oder der Linsenführungsteil eine Aufnahmekammer aufweist, in der eine Intraokularlinse bereits eingelegt ist, sodass der Injektor mit der eingelegten Intraokularlinse eine einzeln handhabbare und handelbare Einheit bildet und die Spezifikationen der eingelegten Linse trägt. Dabei kann das Linsenführungsteil ebenfalls die Aufnahmekammer aufweisen, die auch als Teil des Grundkörpers verstanden werden kann. Die Aufnahmekammer muss insofern nicht örtlich gesehen im Grundkörper eingerichtet sein, und diese kann auch im Linsenführungsteil oder zwischen Linsenführungsteil und Grundkörper eingerichtet sein.

Im Grundkörper ist weiterhin ein Kolben aufgenommen, der über einen Drehübertrager mit dem Betätigungselement axial verschiebbar ist. Insbesondere dann, wenn der Injektor gemäß der Schraubbetätigung bedient werden soll, muss die Drehbewegung im Betätigungselement entkoppelt werden von einer Bewegung eines Kolbens, der insbesondere nur eine Linearbewegung entlang der Betätigungsachse ausführen soll, ohne selbst eine Drehbewegung auszuführen.

Der Grundkörper kann mit dem Aufnahmeabschnitt und mit dem Flügelgriff und/oder insbesondere auch mit dem Linsenführungsteil einteilig und materialeinheitlich aus einem Kunststoffkörper gebildet sein, wobei vorderseitig am Grundkörper der Linsenführungsteil mit einer spitzenseitigen Austriebsdüse zum Austrieb der Intraokularlinse wahlweise auch separat angeordnet sein kann.

### BEVORZUGTES AUSFÜHRUNGSBEISPIEL DER ERFINDUNG

Weitere, die Erfindung verbessernde Maßnahmen werden nachstehend gemeinsam mit der Beschreibung eines bevorzugten Ausführungsbeispiels der Erfindung anhand der Figuren näher dargestellt. Es zeigt:
- Figur 1: eine perspektivische Ansicht eines Injektors zur Implantation einer Intraokularlinse in ein menschliches Auge, wobei das Stellelement des Injektors einer Stellung angeordnet ist, in der der Injektor mit der Schubbetätigung bedient werden kann,
- Figur 2: eine Ansicht des Injektors gemäß Figur 1, wobei das Stellelement des Injektors einer Stellung angeordnet ist, in der der Injektor mit der Schraubbetätigung bedient werden kann,
- Figur 3: eine Explosionsdarstellung der wesentlichen Bestandteile des Injektors,
- Figur 4: eine Querschnittsansicht des Injektors in einer perspektivischen Darstellung, wobei der Betriebszustand des Injektors für die Schubbetätigung eingerichtet ist,
- Figur 5: die Darstellung des Injektors gemäß Figur 4, wobei das Stellelement gegenüber der in Fig. 4 dargestellten Stellung so verdreht wurde, dass die Schraubbetätigung des Injektors eingerichtet ist,
- Figur 6: eine perspektivische Ansicht des Injektors mit einer teilgeschnittenen Ansicht des Stellelementes,
- Figuren 7 und 8: eine Querschnittsansicht durch die Anordnung des Stellelementes auf dem Grundkörper mit einer Schubbetätigung (Figur 7) und mit einer Schraubbetätigung (Figur 8).

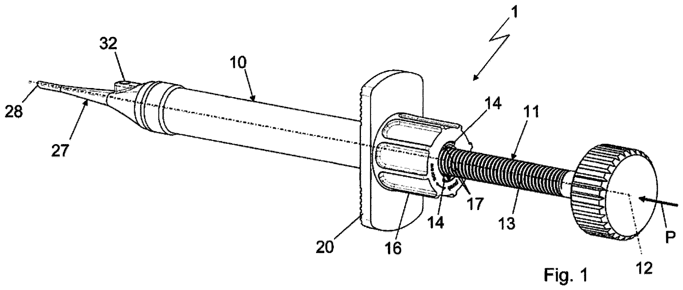

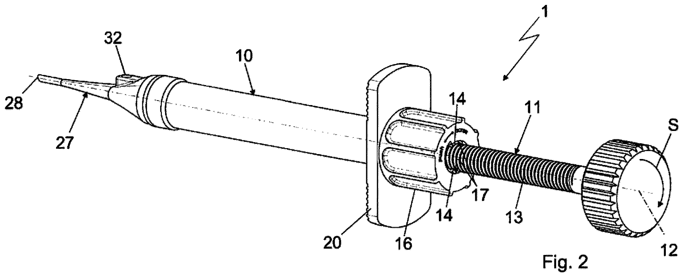

Die Figuren 1 und 2 zeigen eine perspektivische Ansicht des Injektors 1, der zur Implantation einer Intraokularlinse in ein menschliches Auge dient. Der Injektor 1 weist als wesentliches Strukturbauteil einen Grundkörper 10 auf, und in diesen ist rückseitig ein länglich ausgebildetes Betätigungselement 11 mit einem endseitigen Griffstück 31 teilweise hineingeführt. Das Betätigungselement 11 besitzt über seiner wesentlichen Länge einen Abschnitt mit einem Außengewinde 13, und das Betätigungselement 11 ist abschnittsweise rückseitig in den Grundkörper 10 so eingeführt, dass das Griffstück 31 an einem freien Ende des Betätigungselementes 11 ausgebildet ist.

Vorderseitig am Grundkörper 10 ist ein Linsenführungsteil 27 angeordnet, in dem in nicht näher gezeigte Weise die Intraokularlinse aufgenommen ist. Der wesentliche Bestandteil des Grundkörpers 10 ist etwa zylinderförmig oder für die Handhabung ergonomisch für eine menschliche Hand ausgestaltet und weist eine längliche Erstreckung auf, und der Injektor 1 mit dem Grundkörper 10, mit dem vorderseitigen Linsenführungsteil 27 und mit dem rückseitig eingeführten Betätigungselement 11 erstreckt sich länglich in einer Betätigungsachse 12. Die Betätigungsachse 12 bildet dabei zugleich die Rotationsachse für das drehbare Betätigungselement 11 mit dem endseitigen Griffstück 31.

Zur verbesserten Handhabung befindet sich auf dem hinteren Teil des Grundkörpers 10 ein Flügelgriff 20, sodass der Injektor 1 mit dem Flügelgriff 20 zwischen den Zeigefinger und den Mittelfinger genommen werden kann, während mit dem Daumen rückseitig auf das Griffstück 31 gedrückt werden kann. Rückseitig zum Flügelgriff 20 schließt sich ein Stellelement 16 an, das um die Betätigungsachse 12 drehbar am Grundkörper 10 aufgenommen ist, und das Stellelement 16 besitzt einen Durchgang, durch den hindurch sich das Betätigungselement 11 erstreckt. Das Stellelement 16 ist dabei so am Grundkörper 10 eingerichtet, dass dieses keinen direkten Kontakt mit dem Betätigungselement 11 aufweist und mit diesem auch nicht unmittelbar zusammenwirkt.

Am Linsenführungsteil 27 ist eine Eingabeöffnung 32 gezeigt, durch die vor Inbetriebnahme des Injektors ein Viskoelastikum eingegeben werden kann. Dieses benetzt sodann die innenliegende Intraokularlinse und das innere Lumen insbesondere im Linsenführungsteil 27, um den Prozess des Austreibens der Intraokularlinse zu begünstigen beziehungsweise für die Intraokularlinse beschädigungsfrei zu ermöglichen.

Der Injektor 1 weist mit dem Stellelement 16 ein Mittel auf, mit dem für den Gebrauch des Injektors 1 zwischen einer Schubbetätigung P und einer Schraubbetätigung S umgeschaltet werden kann. Figur 1 zeigt dabei eine Stellposition des Stellelementes 16, gemäß der eine Schubbetätigung P möglich ist, während Figur 2 die Stellung des Stellelementes 16 in einer verdrehten Stellung zeigt, in der eine Schraubbetätigung S ermöglicht ist.

Damit das Stellelement 16 ein Mittel zur Umschaltung zwischen der Schubbetätigung P und der Schraubbetätigung S bilden kann, besitzt das Stellelement 16 innenseitig eine Innenoberfläche 17, die sich über dem Umfangsverlauf des Stellelementes 16 nach Art einer archimedischen Spirale zumindest abschnittsweise so im Radius verändert, dass bei einer Verdrehung innenliegende Federarme 14, die ortsfest am Grundkörper 10 angeordnet sind, beim Zuziehen in Richtung zum Außengewinde 13 des Betätigungselementes 11 bewegt und bei einem entgegengesetzten Öffnen von diesem wegbewegt werden können.

So zeigt Figur 1 eine Stellposition des Stellelementes 16, gemäß der sich die Federarme 14 in einem Umfangsbereich an der Innenoberfläche 17 des Stellelementes 16 befinden, in der die Federarme 14 selbsttätig auffedern können, sodass sich der Abstand zur Betätigungsachse 12 vergrößert und sodass die Federarme 14 das Außengewinde 13 auf dem Betätigungselement 11 freigeben.

Hingegen ist in Figur 2 das Stellelement 16 in einer Stellposition verdreht gezeigt, gemäß der die Federarme 14 durch den kleineren Innenradius an dieser Stelle in das Außengewinde 13 des Betätigungselementes 11 gedrückt werden und in dieses Eingreifen, sodass in dieser Position die Schraubbetätigung S für den Vortrieb des Betätigungselementes 11 in den Grundkörper 10 hinein ermöglicht ist.

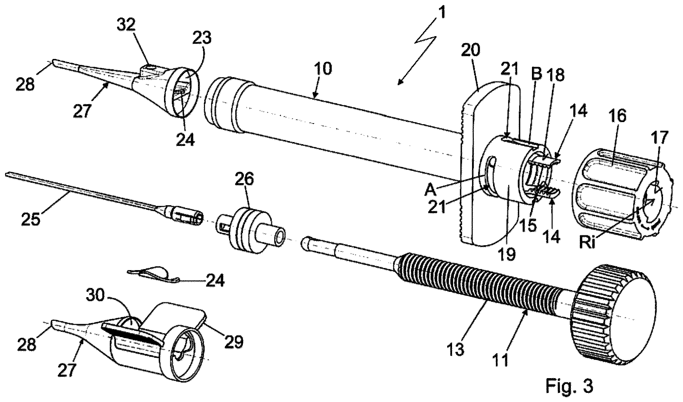

Je weiter das Betätigungselement 11 in den Grundkörper 10 eingedrückt oder eingeschraubt wird, desto weiter wird die Intraokularlinse in Richtung zu einer vorderseitigen Austriebsdüse 28 am Linsenführungsteil 27 nach vorne bewegt. Eine detailliertere Ansicht der Funktion für die Umschaltung des Injektors 1 zwischen der Schubbetätigung P und der Schraubbetätigung S zeigt Figur 3 in einer Explosionsdarstellung des Injektors 1.

Figur 3 zeigt den Injektor 1 in einer Explosionsdarstellung, in der die wesentlichen Bestandteile des Injektors 1 dargestellt sind, wobei weitere Bestandteile des Injektors 1 vorhanden sind, die nicht dargestellt sind, da diese zur Darlegung der vorliegenden Erfindung nicht notwendig sind, die jedoch auch Bestandteil des erfindungsgemäßen Injektors 1 sein sollen, sodass die Abbildung nicht abschließend zu verstehen ist.

Die Darstellung in Figur 3 zeigt den Grundkörper 10 vereinzelt vom Linsenführungsteil 27, und ein fluchtend mit der Betätigungsachse 12 dargestelltes Linsenführungsteil 27 ist mit einer Aufnahmekammer 23 ausgeführt, in der eine Intraokularlinse 24 eingelegt ist (sichtbar mit einer einseitigen Haptik), und ein unterseitig dargestelltes Linsenführungsteil 27 bildet eine alternative Ausgestaltung und weist ein Aufnahmemittel 30 zur Aufnahme einer Linsenkartusche 29 auf, in der die vereinzelt dargestellte Intraokularlinse 24 eingelegt ist. Ist die Linsenkartusche 29 in das Aufnahmemittel 30 eingelegt, kann die Intraokularlinse 24 mit einem Kolben 25 vorderseitig aus der Austriebsdüse 28 in die hintere Augenkammer des menschlichen Auges ausgetrieben werden.

Hierfür führt der Kolben 25 eine Linearbewegung in der Betätigungsachse 12 aus, und wird das Betätigungselement 11 mit einer Schraubbetätigung in den Grundkörper 10 eingedreht, so dient ein Drehübertrager 26 dafür, dass die Rotationsbewegung des Betätigungselementes 11 nicht auf den Kolben 25 übertragen wird.

Hinter dem Flügelgriff 20 besitzt der Grundkörper 10 in Gestalt einer Anformung einen Aufnahmeabschnitt 19, auf den das Stellelement 16 um die Betätigungsachse 12 verdrehbar aufgerastet wird. Hierfür weist die Außenoberfläche des Aufnahmeabschnittes 19 eine Nut 21 auf, die auf dem gezeigten Abschnitt A in Umfangsrichtung verläuft, sodass das Stellelement 16 in einer axial festen Position um die Betätigungsachse 12 verdreht werden kann. Zum Aufschieben dient ein Abschnitt B der Nut 21, wobei eine Rastnase vorhanden ist und überwunden werden muss, sodass das Stellelement 16 in einer Position nicht vom Abschnitt A in den Abschnitt B der Nut 21 vom Aufnahmeabschnitt 19 überwechseln kann.

Der Aufnahmeabschnitt 19 ist hülsenförmig ausgeführt und insbesondere einteilig mit dem Grundkörper 10 rückseits des Flügelgriffes 20 verbunden. Innerhalb des Aufnahmeabschnittes 19 sind beispielhaft von der Anzahl zwei sich gegenüberliegende Federarme 14 angeordnet, wobei die Federarme 14 entweder innenseitig im Aufnahmeabschnitt 18 oder rückseitig am Grundkörper 10 beziehungsweise am Flügelgriff 20 angewurzelt sind. So können die Federarme 14 durch eine elastische Verformung in Richtung zur Betätigungsachse 12 nach innen oder von der Betätigungsachse 12 weg nach außen federnd bewegt werden. In einer nicht kraftbeaufschlagten Anordnung der Federarme 14 weisen diese einen Abstand zueinander auf, gemäß dem das Außengewinde 13 auf dem Betätigungselement 11 mittig zwischen den beiden Federarmen 14 frei bewegt werden kann. Der Auslieferzustand des Injektors 1 sollte daher in einer Stellung des Stellelementes 16 eingerichtet werden, in der dieses in der Stellposition für die Schubbetätigung steht.

Wird das Stellelement 16 auf den Aufnahmeabschnitt 19 aufgerastet, kann das Stellelement 16 auf dem Aufnahmeabschnitt 19 so verdreht werden, dass verschiedene Umfangspositionen der Innenoberfläche 17 mit einem sich ändernden Innenradius Ri in Kontakt mit der Außenfläche 18 der Federarme 14 gelangen. Dadurch, dass sich über den fortlaufenden Umfang der innere Radius Ri der Innenoberfläche 17 verändert, der Radius sich also verjüngt oder erweitert, können die Federarme 14 entweder radial nach innen gedrückt werden oder durch ihre Eigenelastizität selbsttätig wieder nach außen auffedern. Somit kann je nach Stellposition des Stellelementes 16 um die Betätigungsachse 12 eingestellt werden, ob eine Gewindeeingriffsstruktur 15 auf der Innenseite der Federarme 14 in Eingriff mit dem Außengewinde 13 gelangt oder ob die Gewindeeingriffsstruktur 15 außer Eingriff mit dem Außengewinde 13 gebracht wird. Befindet sich die Gewindeeingriffsstruktur 15 an den Federarmen 14 in Eingriff mit dem Außengewinde 13 auf dem Betätigungselement 11, so kann der Injektor 1 mit der Schraubbetätigung S genutzt werden, und befinden sich die Gewindeeingriffsstrukturen 15 auf den Federarmen 14 außer Eingriff mit dem Außengewinde 13 des Betätigungselementes 11, so kann der Injektor 1 mit der Schubbetätigung S genutzt werden.

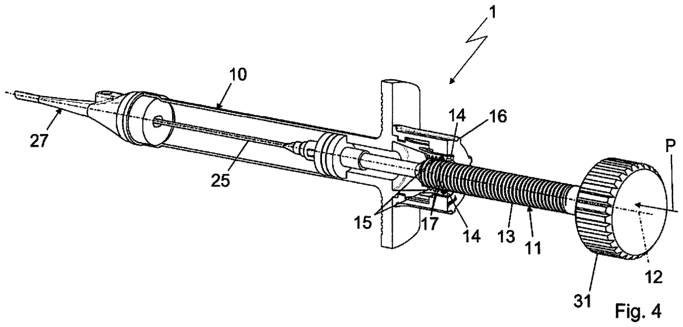

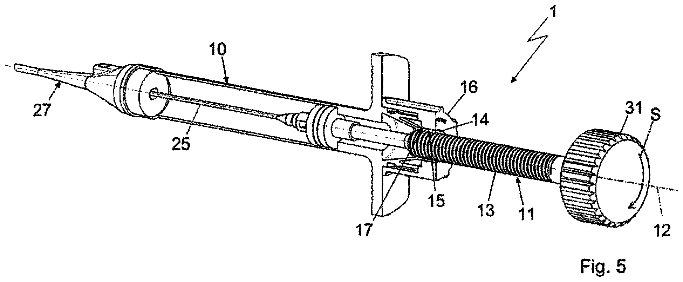

Die Figuren 4 und 5 zeigen weitere perspektivische Ansichten des Injektors 1 in einem Querschnitt durch den Grundkörper 10, wobei zur tieferen Verdeutlichung Figur 4 den Injektor 1 in einem Zustand zeigt, in dem dieser mit der Schubbetätigung P gebraucht werden kann, und Figur 5 zeigt den Injektor 1 in einem Zustand, in dem dieser mit der Schraubbetätigung s gebraucht werden kann.

Soll der Injektor 1 in dem Betriebsmodus der Schubbetätigung P bereitgestellt werden, so wird das Stellelement 16 aus der Blickrichtung des Griffstückes 31 gegen den Uhrzeigersinn verdreht, bis dieses sich in der in Figur 4 gezeigten Drehposition befindet. In dieser Drehposition ist der Innenradius der Innenoberfläche 17 in der Kontaktposition des Federarms 14 so aufgeweitet, dass der Federarm 14 auffedert und einen maximalen Abstand zur Betätigungsachse 12 aufweist. In dieser Position greift die Gewindeeingriffsstruktur 15 auf der Innenseite des Federarms 14 nicht in das Außengewinde 13 des Betätigungselementes 11 ein. Sodann kann das Betätigungselement 11 in der Betätigungsachse 12 frei bewegt werden, und das Betätigungselement 11 kann gemeinsam mit dem Kolben 25 nach vorne in Richtung zum Linsenführungsteil 27 gedrückt werden.

In Figur 5 befindet sich das Stellelement 16 in einer im Uhrzeigersinn verdrehten Position, gemäß der der Innenradius der Innenoberfläche 17 im Kontaktpunkt mit dem Federarm 14 so stark verkleinert ist, dass die Gewindeeingriffsstruktur 15 in Eingriff mit dem Außengewinde 13 auf dem Betätigungselement 11 gebracht ist. In dieser Stellposition des Stellelementes 16 kann das Betätigungselement 11 nur in der Betätigungsachse 12 verlagert werden, wenn dieses vermittels des Griffstückes 31 in der Betätigungsachse 12 in Drehbewegung versetzt wird und schließlich in den Grundkörper 10 eingeschraubt wird. Die sich gegenüberliegenden Gewindeeingriffsstrukturen 15 der Federarme 14 bilden damit einen Gewindedurchgang im Grundkörper 10, über den das Betätigungselement 11 rückseitig in den Grundkörper 10 eingeschraubt werden kann. Nur dann kann der Kolben 25 nach vorne in Richtung zum Linsenführungsteil 27 verlagert werden, und die Intraokularlinse kann aus der Austriebsdüse ausgetrieben werden.

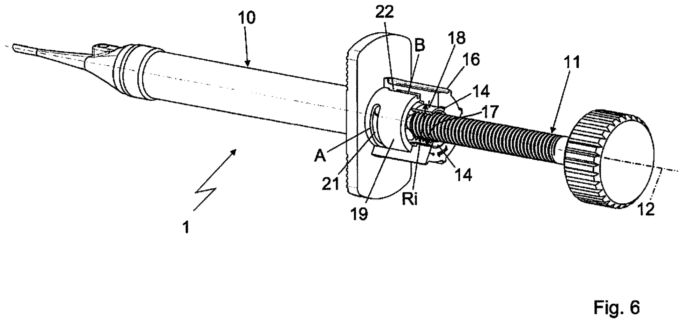

Figur 6 zeigt in einer noch weiteren perspektivischen Ansicht den Injektor 1 mit einem teilweise aufgeschnittenen Stellelement 16, der auf dem Aufnahmeabschnitt 19 aufgerastet ist. Innenseitig im Stellelement 16 sind Zapfen 22 vorhanden, von denen einer mittels des Schnittes sichtbar ist und der in der Nuten 21 verläuft. So kann das Stellelement 16 zunächst über den Abschnitt B der Nut 21 auf den Aufnahmeabschnitt 19 aufgesetzt werden und schließlich innerhalb des Abschnittes A der Nut 21 um die Betätigungsachse 12 verdreht werden.

Die Federarme 14 sind mit rückseitigen Außenflächen 18 so ausgestaltet, dass diese innenseitig entlang der Innenoberfläche 17 des Stellelementes 16 abgleiten können, wenn das Stellelement 16 um die Betätigungsachse 12 verdreht wird. Da die Federarme 14 innenseitig im Aufnahmeabschnitt 19 oder rückseitig am Grundkörper 10 angewurzelt sind, verlagern sich die Federarme 14 nicht in ihrer Umfangsposition um die Betätigungsachse 12. Nur so gelangen die Außenflächen 18 der Federarme 14 bei Verdrehung des Stellelementes 16 mit der Innenfläche 17 in unterschiedlichen Umfangspositionen des Stellelementes 16 in Kontakt, sodass durch den sich ändernden Innenradius Ri die Verlagerung der Federarme 14 zur Betätigungsachse 12 hin und von der Betätigungsachse 12 weg erzeugt werden kann.

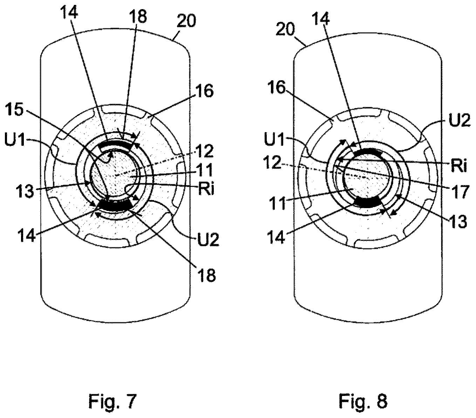

Die Figuren 7 und 8 zeigen eine Querschnittsansicht durch die Anordnung des Stellelementes 16 in Anordnung am Grundkörper, dargestellt durch den Flügelgriff 20. Durch das Stellelement 16 erstreckt sich das Betätigungselement 11 hindurch, und die Betätigungsachse 12 steht senkrecht auf der Ebene der Abbildung.

Figur 7 zeigt die Stellposition des Stellelementes 16, in der die Schubbetätigung ermöglicht ist, und Figur 8 zeigt eine Stellposition des Stellelementes 16, in der die Schraubbetätigung ermöglicht ist.

Zur Einstellung der Schubbetätigung befinden sich die Federarme 14 über ihre außenseitigen Außenflächen 18 an einer Umfangsposition in Kontakt mit der Innenoberfläche 17, in der der Innenradius Ri besonders groß ist. Durch das eigenelastische Auffedern der Federarme 14 gelangt die Gewindeeingriffsstruktur 15 außer Eingriff mit dem Außengewinde 13 auf dem Betätigungselement 11.

Wie in Figur 8 gezeigt, kann das Stellelement 16 mit dem Uhrzeigersinn in eine Stellposition verdreht werden, in der die Schraubbetätigung S ermöglicht ist. In dieser Position befindet sich die Außenfläche 18 des Federarms 14 an einer Umfangsposition in Kontakt mit der Innenoberfläche 17, in der der Innenradius Ri kleiner ist, und die Gewindeeingriffsstruktur 15 innenseitig in den Federarmen 14 wird in Eingriff mit dem Außengewinde 13 auf dem Betätigungselement 11 gebracht. In dieser Position kann das Betätigungselement 11 nur durch eine Schraubbewegung in den Grundkörper eingeschraubt werden.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch macht. Sämtliche aus den Ansprüchen, der Beschreibung oder den Zeichnungen hervorgehenden Merkmale und/oder Vorteile, einschließlich konstruktiver Einzelheiten oder räumlicher Anordnungen, können sowohl für sich als auch in den verschiedensten Kombinationen erfindungswesentlich sein.

### Bezugszeichenliste:

- 1: Injektor
- 10: Grundkörper
- 11: Betätigungselement
- 12: Betätigungsachse
- 13: Außengewinde
- 14: Federarm
- 15: Gewindeeingriffsstruktur
- 16: Stellelement
- 17: Innenoberfläche
- 18: Außenfläche
- 19: Aufnahmeabschnitt
- 20: Flügelgriff
- 21: Nut
- 22: Zapfen
- 23: Aufnahmekammer
- 24: Intraokularlinse
- 25: Kolben
- 26: Drehübedrager
- 27: Linsenführungsteil
- 28: Austriebsdüse
- 29: Linsenkartusche
- 30: Aufnahmemittel
- 31: Griffstück
- 32: Eingabeöffnung
- A: Abschnitt der Nut
- B: Abschnitt der Nut
- Ri: Innenradius
- P: Schubbetätigung
- S: Schraubbetätigung

## Patentansprüche

1. Injektor (1) zur Implantation einer Intraokularlinse, aufweisend einen Grundkörper (10), in den rückseitig ein länglich ausgebildetes Betätigungselement (11) abschnittsweise hineinragt und das in einer Betätigungsachse (12) beweglich geführt ist, und wobei eine Bewegung des Betätigungselementes (11) in den Grundkörper (10) wahlweise mittels einer Schubbetätigung (P) entlang der Betätigungsachse (12) oder mittels einer Schraubbetätigung (S) um die Betätigungsachse (12) erzeugbar ist, wobei das Betätigungselement (11) wenigstens auf einem Abschnitt ein Außengewinde (13) aufweist, und wobei am Grundkörper (10) ein um die Betätigungsachse (12) drehbares Stellelement (16) mit einem Durchgang angeordnet ist, durch den sich das Betätigungselement (11) hindurch erstreckt,
**dadurch gekennzeichnet,**
**dass** am Grundkörper (10) wenigstens ein Federarm (14) angeordnet ist, der an einer zum Außengewinde (13) weisenden Innenfläche eine Gewindeeingriffsstruktur (15) aufweist, , wobei der Durchgang des Stellelementes (16) wenigstens eine Innenoberfläche (17) aufweist, die in einer Umfangsrichtung wenigstens abschnittsweise einen sich ändernden Innenradius (Ri) aufweist, und wobei eine Außenfläche (18) des Federarms (14) mit der Innenoberfläche (17) des Stellelementes (16) in Kontakt steht, sodass bei einer Verdrehung des Stellelementes (16) um die Betätigungsachse (12) aufgrund des sich ändernden Innenradius (Ri) an der Umfangsposition des Federarmes (14) eine elastische Verformung des Federarms (14) zur Betätigungsachse (12) hin und von der Betätigungsachse (12) weg erzeugbar ist, wodurch die Gewindeeingriffsstruktur (15) mit dem Außengewinde (13) wahlweise in Eingriff oder außer Eingriff bringbar ist.

2. Injektor (1) nach Anspruch 1,
**dadurch gekennzeichnet,**
**dass** der Grundkörper (10) einen Aufnahmeabschnitt (19) mit einem Durchgang aufweist, durch den sich das Betätigungselement (11) hindurch erstreckt und auf den das Stellelement (16) verdrehbar aufgenommen ist, wobei der Grundkörper (10) einen Flügelgriff (20) aufweist, wobei sich der Aufnahmeabschnitt (19) rückseitig des Flügelgriffs (20) als Fortsatz anschließt.

3. Injektor (1) nach Anspruch 1 oder 2,
**dadurch gekennzeichnet,**
**dass** der wenigstens eine Federarm (14) endseitig am oder innerhalb des Aufnahmeabschnittes (19) am Aufnahmeabschnitt (19) oder am Grundkörper (10) angeordnet ist.

4. Injektor (1) nach einem der Ansprüche 1 oder 3,
**dadurch gekennzeichnet,**
**dass** das Stellelement (16) auf den Aufnahmeabschnitt (19) aufgerastet und in einer mit Bezug auf die Betätigungsachse (12) festen Axialposition auf dem Aufnahmeabschnitt (19) verdrehbar geführt ist.

5. Injektor (1) nach einem der Ansprüche 2 bis 4,
**dadurch gekennzeichnet,**
**dass** im Außenumfang des Aufnahmeabschnittes (19) wenigstens eine Nut (21) eingebracht ist, und wobei innenseitig im Stellelement (16) wenigstens ein Zapfen (22) ausgebildet ist, der in der Nut (21) geführt ist.

6. Injektor (1) nach Anspruch 5,
**dadurch gekennzeichnet,**
**dass** die Nut (21) wenigstens auf einem Abschnitt (A) in Umfangsrichtung der Außenoberfläche des Aufnahmeabschnittes (19) verläuft.

7. Injektor (1) nach einem der vorgenannten Ansprüche,
**dadurch gekennzeichnet,**
**dass** die Innenoberfläche (17) mit dem sich in Umfangsrichtung ändernden Innenradius (Ri) auf einem Umfangssegment (U1, U2) des Stellelements (16) ausgebildet ist.

8. Injektor (1) nach Anspruch 7,
**dadurch gekennzeichnet,**
**dass** das Umfangssegment (U1, U2) sich auf einem Umfang mit einem Umfangswinkel von 360° oder auf einem Umfangswinkel von 180° oder auf einem Umfangswinkel von 120° oder auf einem Umfangswinkel von 90° erstreckt.

9. Injektor (1) nach Anspruch 7,
**dadurch gekennzeichnet,**
**dass** zwei sich gegenüberliegende Umfangssegmente (U1, U2) in der Innenoberfläche (17) des Stellelementes (16) ausgebildet sind, und wobei zwei sich gegenüberliegende Federarme (14) am Grundkörper (10) und/oder am Aufnahmeabschnitt (19) angeordnet sind, die jeweils einem Umfangssegment (U1, U2) zugeordnet sind und mit diesen zusammenwirken.

10. Injektor (1) nach einem der vorgenannten Ansprüche,
**dadurch gekennzeichnet,**
**dass** das Außengewinde (13) auf dem Betätigungselement (11) eine Steigung von 3mm bis 20mm aufweist.

11. Injektor (1) nach einem der vorgenannten Ansprüche,
**dadurch gekennzeichnet,**
**dass** der Injektor (1) ein Aufnahmemittel (30) für eine Linsenkartusche (29) aufweist, in der eine Intraokularlinse (24) eingebracht ist und wobei die Linsenkartusche (29) in das Aufnahmemittel (30) einsetzbar ist.

12. Injektor (1) nach einem der Ansprüche 1 bis 10,
**dadurch gekennzeichnet,**
**dass** der Grundkörper (10) eine Aufnahmekammer (23) aufweist, in der eine Intraokularlinse (24) eingelegt ist, sodass der Injektor (1) mit der eingelegten Intraokularlinse (24) eine einzeln handhabbare und handelbare Einheit bildet.

13. Injektor (1) nach einem der vorgenannten Ansprüche,
**dadurch gekennzeichnet,**
**dass** im Grundkörper (10) ein Kolben (25) aufgenommen ist, der über einen Drehübertrager (26) mit dem Betätigungselement (11) axial verschiebbar ist.

14. Injektor (1) nach Anspruch 2,
**dadurch gekennzeichnet,**
**dass** der Grundkörper (10) mit dem Aufnahmeabschnitt (19) und mit dem Flügelgriff (20) einteilig aus einem Kunststoffkörper gebildet ist, wobei vorderseitig am Grundkörper (10) ein Linsenführungsteil (27) mit einer spitzenseitigen Austriebsdüse (28) zum Austrieb der Intraokularlinse (24) angeordnet ist.

## Claims

1. Injector (1) for implantation of an intraocular lens (24), having a base body (10) and an elongated actuation element (11), which projects at least partly into a rear section of the base body (10) and which is movably guided in an actuation axis (12), and wherein a movement of the actuation element (11) into the base body (10) can be generated selectively by means of a push actuation (P) along the actuation axis (12) or by means of a screw actuation (S) about the actuation axis (12), and wherein the actuation element (11) features an external thread (13) at least on one section, wherein a switch element (16), which can be rotated about the actuation axis (12), is arranged on the base body (10) and has a passage, through which the actuation element (11) extends,
**characterized in,**
**that** at least one spring arm (14) is arranged at the base body (10), wherein the spring arm (14) has a thread engagement structure (15) on an inner surface facing the external thread (13), and wherein the passage of the switch element (16) features at least one inner surface (17) having an inner radius (Ri) varying at least in sections in a circumferential direction, and wherein an outer surface (18) of the spring arm (14) is in contact with the inner surface (17) of the switch element (16), so that upon a rotation of the switch element (16) about the actuation axis (12), due to the varying inner radius (Ri) at the circumferential position of the spring arm (14), an elastic deformation of the spring arm (14) towards the actuation axis (12) and away from the actuation axis (12) can be generated, whereby the thread engagement structure (15) can be selectively engaged or disengaged with the external thread (13).

2. Injector (1) according to claim 1,
**characterized,**
**in that** the base body (10) has a receiving section (19) with a passage, through which the actuation element (11) extends and onto which the switch element (16) is rotatably received, whereas the base body (10) has a wing handle (20), wherein the receiving section (19) adjoins the rear side of the wing handle (20) as an extension.

3. Injector (1) according to claim 1 or 2,
**characterized,**
**in that** the at least one spring arm (14) is arranged to the receiving section (19) in a position at the end side or within the receiving section (19).

4. Injector (1) according to one of claims 1 or 3,
**characterized,**
**in that** the switch element (16) is snapped onto the receiving section (19) and is guided rotatably on the receiving section (19) in a certain axial position with respect to the actuation axis (12).

5. Injector (1) according to one of claims 2 to 4,
**characterized,**
**in that** at least one groove (21) is provided in the outer circumference of the receiving section (19), and wherein at least one pin (22) is formed on the inside of the switch element (16), wherein the pin (22) is guided in the groove (21).

6. Injector (1) according to claim 5,
**characterized,**
**in that** the groove (21) runs at least on one section (A) in the circumferential direction of the outer surface of the receiving section (19).

7. Injector (1) according to one of the aforementioned claims,
**characterized,**
**in that** the inner surface (17) with the inner radius (Ri) varying in the circumferential direction is formed on a circumferential segment (U1, U2) of the switch element (16).

8. Injector (1) according to claim 7,
**characterized,**
**in that** the circumferential segment (U1, U2) extends on a circumference with a circumferential angle of 360° or with a circumferential angle of 180° or with a circumferential angle of 120° or with a circumferential angle of 90°.

9. Injector (1) according to claim 7,
**characterized,**
**in that** two opposite circumferential segments (U1, U2) are formed in the inner surface (17) of the switch element (16), and wherein two opposite spring arms (14) are arranged at the base body (10) and/or at the receiving section (19), wherein the spring arms (14) are each assigned to a circumferential segment (U1, U2) and interact with the latter.

10. Injector (1) according to one of the aforementioned claims,
**characterized,**
**in that** the external thread (13) on the actuation element (11) has a thread pitch of 3mm to 20mm.

11. Injector (1) according to one of the aforementioned claims,
**characterized,**
**in that** the injector (1) features a receiving means (30) for a lens cartridge (29), in which an intraocular lens (24) is inserted, and wherein the lens cartridge (29) can be inserted into the receiving means (30).

12. Injector (1) according to one of claims 1 to 10,
**characterized,**
**in that** the base body (10) features a receiving chamber (23), in which an intraocular lens (24) is inserted, so that the injector (1) forms an individually manageable and tradable unit together with the inserted intraocular lens (24).

13. Injector (1) according to one of the aforementioned claims,
**characterized,**
**in that** a piston (25) is received in the base body (10) and can be displaced axially by the actuation element (11) via a rotary joint (26).

14. Injector (1) according to one of the aforementioned claims,
**characterized,**
**in that** the base body (10) is formed in one piece with the receiving section (19) and with the wing handle (20) from a plastic body, wherein a lens guide section (27) with a tip-side ejection nozzle (28) for ejecting the intraocular lens (24) is arranged at the front end of the base body (10).

## Revendications

1. Injecteur (1) destiné à l'implantation d'une lentille intraoculaire, présentant un corps de base (10), dans lequel, sur le côté arrière, un élément d'actionnement (11) réalisé allongé dépasse en partie et est guidé mobile dans un axe d'actionnement (12), et dans lequel un déplacement de l'élément d'actionnement (11) dans le corps de base (10) peut être généré au choix au moyen d'un actionnement par coulissement (P) le long de l'axe d'actionnement (12) ou au moyen d'un actionnement par vissage (S) autour de l'axe d'actionnement (12), dans lequel l'élément d'actionnement (11) présente, au moins sur une partie, un filetage extérieur (13), et dans lequel un élément de réglage (16) rotatif autour de l'axe d'actionnement (12) et doté d'un passage est agencé sur le corps de base (10), l'élément d'actionnement (11) s'étendant à travers ledit passage,
**caractérisé en ce que**
au moins un bras ressort (14) est agencé sur le corps de base (10), ledit bras ressort présentant, sur une face interne orientée vers le filetage extérieur (13), une structure de mise en prise par vissage (15), le passage de l'élément de réglage (16) présentant au moins une surface interne (17), qui présente un rayon intérieur (Ri) variable au moins sur certaines parties dans une direction circonférentielle, et une face externe (18) du bras ressort (14) étant en contact avec la surface interne (17) de l'élément de réglage (16), de telle sorte que lors d'une rotation de l'élément de réglage (16) autour de l'axe d'actionnement (12), une déformation élastique du bras ressort (14) vers l'axe d'actionnement (12) et à l'opposé de l'axe d'actionnement (12) peut être générée en raison du rayon intérieur (Ri) variable au niveau de la position circonférentielle du bras ressort (14), moyennant quoi la structure de mise en prise par vissage (15) peut au choix être amenée en prise avec le filetage extérieur (13) ou séparée de celui-ci.

2. Injecteur (1) selon la revendication 1,
**caractérisé en ce que**
le corps de base (10) présente une partie de réception (19) dotée d'un passage à travers lequel s'étend l'élément d'actionnement (11) et sur lequel l'élément de réglage (16) est reçu de manière rotative, le corps de base (10) présentant une poignée papillon (20), la partie de réception (19) étant dans le prolongement de la poignée papillon (20) à l'arrière sous la forme d'un appendice.

3. Injecteur (1) selon la revendication 1 ou 2,
**caractérisé en ce que**
l'au moins un bras ressort (14) est agencé côté extrémité sur ou à l'intérieur de la partie de réception (19) sur la partie de réception (19) ou sur le corps de base (10).

4. Injecteur (1) selon l'une des revendications 1 ou 3,
**caractérisé en ce que**
l'élément de réglage (16) est guidé encliqueté sur la partie de réception (19) et guidé rotatif sur la partie de réception (19) dans une position axiale fixe par rapport à l'axe d'actionnement (12).

5. Injecteur (1) selon l'une des revendications 2 à 4,
**caractérisé en ce que**
au moins une rainure (21) est pratiquée dans la circonférence extérieure de la partie de réception (19), et dans lequel au moins un ergot (22), qui est guidé dans la rainure (21), est réalisé sur le côté intérieur de l'élément de réglage (16).

6. Injecteur (1) selon la revendication 5,
**caractérisé en ce que**
la rainure (21) s'étend au moins sur une partie (A) dans la direction circonférentielle de la surface externe de la partie de réception (19).

7. Injecteur (1) selon l'une des revendications précédentes,
**caractérisé en ce que**
la surface interne (17) avec le rayon intérieur (Ri) variable dans la direction circonférentielle est réalisée sur un segment de circonférence (U1, U2) de l'élément de réglage (16).

8. Injecteur (1) selon la revendication 7,
**caractérisé en ce que**
le segment de circonférence (U1, U2) s'étend sur une circonférence avec un angle inscrit de 360° ou sur un angle inscrit de 180° ou sur un angle inscrit de 120° ou sur un angle inscrit de 90°.

9. Injecteur (1) selon la revendication 7,
**caractérisé en ce que**
deux segments de circonférence (U1, U2) opposés l'un à l'autre sont réalisés dans la surface interne (17) de l'élément de réglage (16) et dans lequel deux bras ressorts (14) opposés l'un à l'autre sont agencés sur le corps de base (10) et/ou sur la partie de réception (19), chacun étant associé à un segment de circonférence (U1, U2) et coopérant avec celui-ci.

10. Injecteur (1) selon l'une des revendications précédentes,
**caractérisé en ce que**
le filetage extérieur (13) sur l'élément d'actionnement (11) présente un pas de 3 mm à 20 mm.

11. Injecteur (1) selon l'une des revendications précédentes,
**caractérisé en ce que**
l'injecteur (1) présente un moyen de réception (30) pour une cartouche de lentille (29), dans laquelle se trouve une lentille intraoculaire (24) et dans lequel la cartouche de lentille (29) peut être introduite dans le moyen de réception (30).

12. Injecteur (1) selon l'une des revendications 1 à 10,
**caractérisé en ce que**
le corps de base (10) présente une chambre de réception (23) dans laquelle une lentille intraoculaire (24) est insérée, de telle sorte que l'injecteur (1) forme, avec la lentille intraoculaire (24) insérée, une unité pouvant être commercialisée et manipulée individuellement.

13. Injecteur (1) selon l'une des revendications précédentes,
**caractérisé en ce que**
un piston (25) est reçu dans le corps de base (10), ledit piston pouvant être déplacé axialement avec l'élément d'actionnement (11) par le biais d'un transmetteur rotatif (26).

14. Injecteur (1) selon la revendication 2,
**caractérisé en ce que**
le corps de base (10) est formé avec la partie de réception (19) et avec la poignée papillon (20) d'un seul tenant en un corps en matière plastique, une pièce de guidage de lentille (27), dotée d'une buse d'expulsion (28) du côté de la pointe destinée à l'expulsion de la lentille intraoculaire (24), étant agencée à l'avant sur le corps de base (10).
